# EUROPEAN PATENT APPLICATION

(11) **EP 4 749 331 A1**
(43) Date of publication of application: **27.05.2026**
(21) Application number: 25209104.6
(22) Date of filing: 16.10.2025
(51) Int. Cl.: G01T 1/185

(54) **METAL WINDOW ION CHAMBERS AND RADIATION APPARATUSES INCLUDING THE SAME**

(30) Priority: 22.11.2024 US 202418956194
(71) Applicant: Varian Medical Systems, Inc., Palo Alto, CA 94304 (US)
(72) Inventor: PLANT, Alex, Mountain View, 94041 (US); POEHLMANN-MARTINS, Flavio, Fremont, 94536 (US); CHEROM KHEIRABADI, Ali, San Francisco, 94102 (US)
(74) Representative: Mathisen & Macara LLP

(57) **Abstract**

An ion chamber (116) comprises a first ion chamber window assembly (200) and an ion chamber housing (1000) having a first opening. The first ion chamber window assembly includes a first ion chamber window (2000) and a first metal plating (200) arranged at a peripheral edge of the first ion chamber window, wherein the first ion chamber window is formed of at least a first metal and has a first surface exposed through the first opening. The first metal plating is formed of a second metal. The first metal is different from the second metal, and the first ion chamber window assembly is fixed, via the first metal plating, on an inner side of the ion chamber housing (1000) at a periphery of the first opening.

## Description

### FIELD

One or more example embodiments relate to ionization (ion) chambers and radiation apparatuses including the same.

### BACKGROUND

Radiation apparatuses include monitor ionization (ion) chambers to measure, for example, the dose distribution profile of a radiation beam delivered by the apparatus. Conventionally, ion chambers include windows and electrodes formed of a polyimide film, such as a Kapton^{®} polyimide film.

### SUMMARY

The scope of protection sought for various example embodiments is set out by the independent claims. The example embodiments and/or features, if any, described in this specification that do not fall under the scope of the independent claims are to be interpreted as examples useful for understanding various embodiments.

Independent of the grammatical term usage, individuals with male, female or other gender identities are included within the term.

At least one example embodiment provides an ion chamber comprising: a first ion chamber window assembly and an ion chamber housing having a first opening. The first ion chamber window assembly includes a first ion chamber window and a first metal plating arranged at a peripheral edge of the first ion chamber window. The first ion chamber window is formed of at least a first metal and has a first surface exposed through the first opening. The first metal plating is formed of a second metal, which is different from the first metal. The first ion chamber window assembly is fixed, via the first metal plating, on an inner side of the ion chamber housing at a periphery of the first opening. The ion chamber is suitably a metal window ion chamber. The ion chamber housing may be entirely or partly copper plated metal, such as copper plated aluminum or copper plated stainless steel. The housing may be hermetically sealed. Alternatively, the housing may be non-hermetically sealed. The ion chamber housing provide an enclosed volume for a gas. The ion chamber housing provide an enclosed volume for internal components including, for example, electrode assemblies, clamping assemblies, support structures, and one or more ion chamber window assemblies.

At least one other example embodiment provides a radiation apparatus comprising: a radiation source configured to emit a radiation beam toward a patient; a radiation beam adjustment structure configured to adjust one or more characteristics of the radiation beam; and an ion chamber arranged in a path of the radiation beam, the ion chamber being configured to monitor one or more characteristics of the radiation beam. The ion chamber includes: a first ion chamber window assembly and an ion chamber housing having a first opening. The first ion chamber window assembly includes a first ion chamber window and a first metal plating arranged at a peripheral edge of the first ion chamber window. The first ion chamber window is formed of at least a first metal and has a first surface exposed through the first opening. The first metal plating is formed of a second metal, which is different from the first metal. The first ion chamber window assembly is fixed, via the first metal plating, on an inner side of the ion chamber housing at a periphery of the first opening. The ion chamber is suitably a metal window ion chamber. The ion chamber may include a housing providing an enclosed volume for a gas. The ion chamber may include spaced electrodes. The radiation apparatus may also include a radiation beam adjustment structure including various collimating devices or components configured to limit, define, and/or modify the size, shape, fluence, and other characteristics of the beam. For example, the radiation beam adjustment structure includes a primary collimator adjacent to the radiation source and (optionally) a secondary collimator to generally limit the extent of the divergence of the radiation beam as the beam travels away from the radiation source. The radiation beam adjustment structure may further include a multileaf collimator (MLC). The radiation beam adjustment structure may further include a flattening filter. The radiation apparatus may further include a metal window ion chamber arranged in the beam path between the flattening filter and the secondary collimator. A metal window ion chamber is suitably arranged in the beam path between a flattening filter and a secondary collimator. The radiation apparatus may be configured to measure and/or analyze signals derived from ion pairs generated by ionization of gas in the ion chamber by radiation using electrical circuitry and use the measurements and/or analysis to control the operation of the radiation apparatus.

According to one or more example embodiments, the first metal may be aluminum, such as 5052 H19 aluminum. The ion chamber window may be about 50 um (0.0020 inches) thick. The ion chamber window, prior to being plated, may be laser cut to avoid deformation (e.g., wrinkling) of the aluminum or other thin metal foil utilized to form the ion chamber window.

The second metal may be nickel, copper, tin, gold or any other readily solderable metal. The first metal plating may be formed of nickel plating, copper plating, tin plating, gold plating, or any other readily solderable metal. In at least one example embodiment, nickel sulfamate (Ni(SO₃NH₂)₂) may be used to create a layer of pure nickel as the metal plating. The metal plating may be formed to a thickness of about 0.007- 0.010 mm (300-400 micro inches). The first metal plating may be formed only at the peripheral edge of the first ion chamber window and/or the first metal plating may be formed outside a beam path through the ion chamber.

The first metal plating may be soldered to the inner side of the ion chamber housing.

According to one or more example embodiments, the ion chamber housing may include a first part having the first opening and a second part having a second opening, wherein the first part and the second part are configured to be fixed to one another to form the ion chamber housing. The first opening and the second opening may be at opposite ends of the ion chamber, and the first ion chamber window assembly may be fixed to an inner side of the first part of the ion chamber housing. The ion chamber may include a second ion chamber window assembly fixed on an inner side of the second part of the ion chamber housing. A second surface of the second ion chamber window assembly may be exposed through the second opening. The metal plating may be ring shaped with an opening in the central portion of the ring. The ion chamber window assembly may be fixed across the opening in the metal plating.

The second ion chamber window assembly may include a second ion chamber window and a second metal plating arranged at a peripheral edge of the second ion chamber window. The second ion chamber window may be formed of at least the first metal, the second metal plating may be formed of the second metal, and the second ion chamber window assembly may be fixed, via the second metal plating, on the inner side of the second part of the ion chamber housing.

The ion chamber may further include: a third ion chamber window assembly arranged within the ion chamber housing between the first ion chamber window assembly and the second ion chamber window assembly; and a window support structure fixed to an inner part of the ion chamber housing. The window support structure may be configured to support the third ion chamber window assembly.

The third ion chamber window assembly may include a third ion chamber window and a third metal plating at a peripheral edge of the third ion chamber window. The third ion chamber window may be formed of at least the first metal, and the third metal plating may be formed of the second metal. The third metal plating may be soldered to the window support structure.

The ion chamber may further include: a first electrode assembly arranged between the first ion chamber window assembly and the third ion chamber window assembly; and a second electrode assembly arranged between the second ion chamber window assembly and the third ion chamber window assembly. The third ion chamber window assembly may be between the first electrode assembly and the second electrode assembly. Each of the first electrode assembly and the second electrode assembly may include a high voltage electrode and a measurement or collection electrode spaced apart from each other by a spacer ring. The collection electrode may include a pattern of a conductive material formed or deposited on an insulative film or plate. For example, the collection electrode may include a combination of electrodes patterned, segmented, or arranged for measuring different combinations of radiation passing through the ion chamber, thereby providing information about the profile of the radiation such as the intensity, the homogeneity of the beam across the treatment filed, the symmetry of the beam about the beam's central axis, the alignment and/or directionality of the beam, and similar.

### BRIEF DESCRIPTION OF THE DRAWINGS

Example embodiments will become more fully understood from the detailed description given herein below and the accompanying drawings, wherein like elements are represented by like reference numerals, which are given by way of illustration only and thus are not limiting of this disclosure.
FIG. 1 is a block diagram illustrating a radiation apparatus including a metal window ion chamber according to an example embodiment.
FIG. 2A is a plan view of a target side of a metal window ion chamber, according to an example embodiment.
FIG. 2B is a plan view of a patient side of the metal window ion chamber shown in FIG. 2A.
FIG. 2C is a patient side perspective view of the metal window ion chamber shown in FIGS. 2A and 2B.
FIG. 3 is a cross-sectional view of the metal window ion chamber shown in FIGS. 2A-2C.
FIG. 4 is a perspective view of an inside of the target side body part shown in FIG. 2A.
FIG. 5 is a perspective view of an inside of the patient side body part shown in FIG. 2B.
FIG. 6 is a perspective view of an ion chamber window assembly according to an example embodiment.

It should be noted that these figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment and should not be interpreted as defining or limiting the range of values or properties encompassed by example embodiments. The use of similar or identical reference numbers in the various drawings is intended to indicate the presence of a similar or identical element or feature.

### DETAILED DESCRIPTION

Various example embodiments will now be described more fully with reference to the accompanying drawings in which some example embodiments are shown.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

It should be understood that there is no intent to limit example embodiments to the particular forms disclosed. On the contrary, example embodiments are to cover all modifications, equivalents, and alternatives falling within the scope of this disclosure. Like numbers refer to like elements throughout the description of the figures.

As discussed herein the terminology "one or more" and "at least one" may be used interchangeably.

It will be appreciated that a number of example embodiments may be used in combination.

One or more example embodiments may be described herein with regard to "upper" and "lower" parts and/or "target side" and "patient side" parts. It should be understood, however, that this terminology is used for example purposes only. In this regard, the "upper" part may be a "lower" part (and vice versa), and a "patient side" part may be a "target side" part (and vice versa), depending on orientation and/or implementation.

Ionization (ion) chambers, according to example embodiments, will be described herein in conjunction with a radiation system or apparatus. It should be noted, however, that while various example embodiments are described in conjunction with a radiation system or apparatus for medical treatment, the scope of the disclosure or of the claims should not be so limited. Ion chambers and the principles described herein may be employed in other applications or industries such as environmental monitoring, research laboratory, etc.

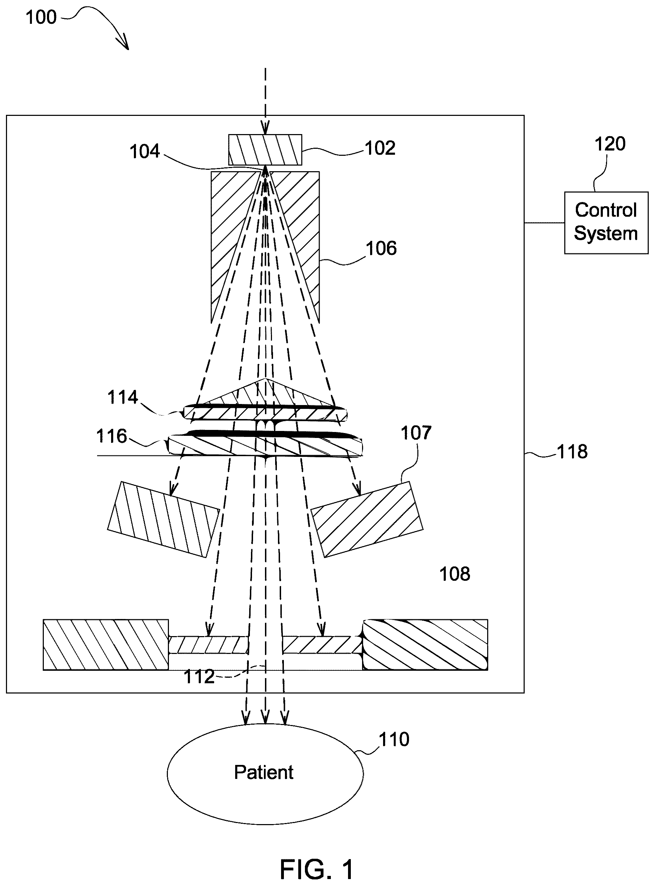

FIG. 1 illustrates a radiation apparatus (also referred to as a radiation therapy treatment apparatus or system) including a metal window ion chamber (also referred to more simply as an ion chamber), according to an example embodiment.

Referring to FIG. 1, the radiation apparatus 100 includes a radiation source 102 configured to produce or emit a beam 104, 112 of radiation such as photons, electrons, protons, or other types of radiation. By way of example, the radiation source 102 may include a metallic target configured to produce a beam of photons or x-rays upon impingement of electrons.

The radiation apparatus 100 also includes a radiation beam adjustment structure (106, 107, 108, 114) including various collimating devices or components configured to limit, define, and/or modify the size, shape, fluence, and other characteristics of the beam. For example, the radiation beam adjustment structure includes a primary collimator 106 adjacent to the radiation source 102 and (optionally) a secondary collimator 107 to generally limit the extent of the divergence of the radiation beam 104, 112 as the beam travels away from the radiation source 102.

The radiation beam adjustment structure further includes a multileaf collimator (MLC) 108 and a flattening filter 114. The MLC 108 is between the radiation source 102 and a patient 110 to shape the beam. The MLC 108 may be rotated about the central axis of the beam 112, which passes through the radiation source 102 and is perpendicular to an isocenter plane, placing the MLC 108 in various orientations.

The flattening filter 114 is positioned in the beam path between first collimator 106 and the secondary collimator 107 to modify the beam profile. In other example embodiments, the flattening filter 114 may be omitted or the radiation apparatus 100 may be flattening-filter-free (FFF) to enhance dose rates for treatment.

Still referring to FIG. 1, the radiation apparatus 100 further includes a metal window ion chamber 116 arranged in the beam path between the flattening filter 114 and the secondary collimator 107. In at least one example embodiment, the ion chamber 116 is configured to monitor one or more characteristics of the beam 104, 112 from the radiation source 102. In more detail, for example, the ion chamber 116 may be configured to measure the dose distribution profile of the beam 104, 112, such as the homogeneity of the beam across the treatment field, the symmetry of the beam about the beam's central axis, the alignment and/or directionality of the beam, and so on. As will be described in greater detail below, the ion chamber 116 may include, among other things, a housing providing an enclosed volume for a gas, spaced electrodes and metal, rather than polyimide, windows. In operation, the gas in the ion chamber 116 is ionized by radiation to produce ion pairs. The spaced electrodes create an electric field allowing the ion pairs to migrate under the influence of the field. Signals derived from the ion pairs, which are directly proportional to the radiation intensity, may be measured and/or analyzed by electrical circuitry and used in controlling the operation of the radiation apparatus 100.

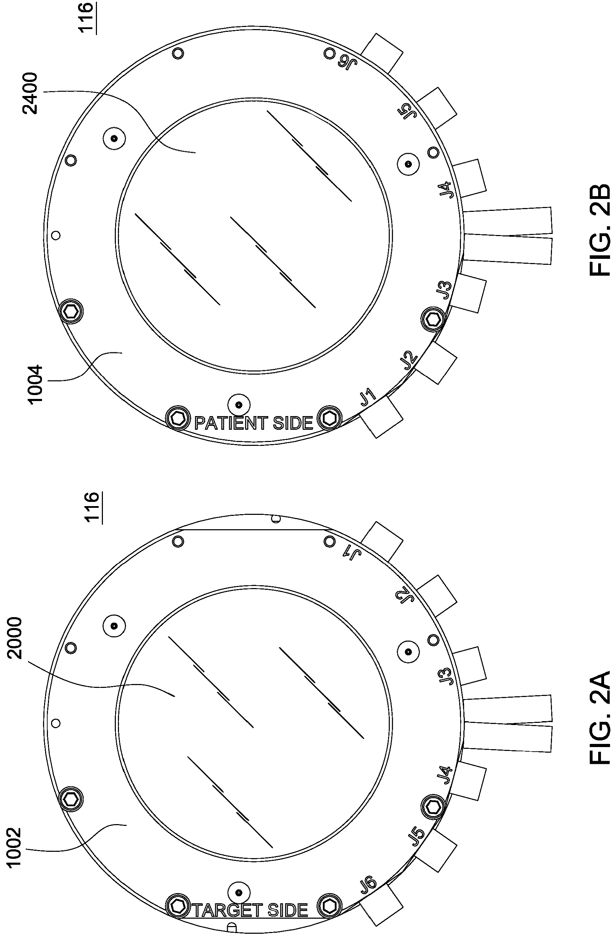

Structures of metal window ion chambers, according to example embodiments, will be discussed in more detail below with regard to FIGS. 2A-6.

Still referring to FIG. 1, the radiation source 102, primary collimator 106, secondary collimators 107, MLC 108, ion chamber 116, and other devices or components may be enclosed in a gantry 118, such as a ring gantry or a C-arm gantry, which may be rotated about an axis such as a horizontal axis, or in an enclosure movable by a robotic arm. Therefore, the radiation apparatus 100 may deliver radiation to a target in the patient 110 from various angles, and one or more characteristics of the radiation beam 104 may be monitored as the beam angle is stepped or swept around the target. The radiation apparatus 100 further includes a control system 120 configured to control operation of the radiation source 102, ion chamber 116, and/or other components of the radiation apparatus 100.

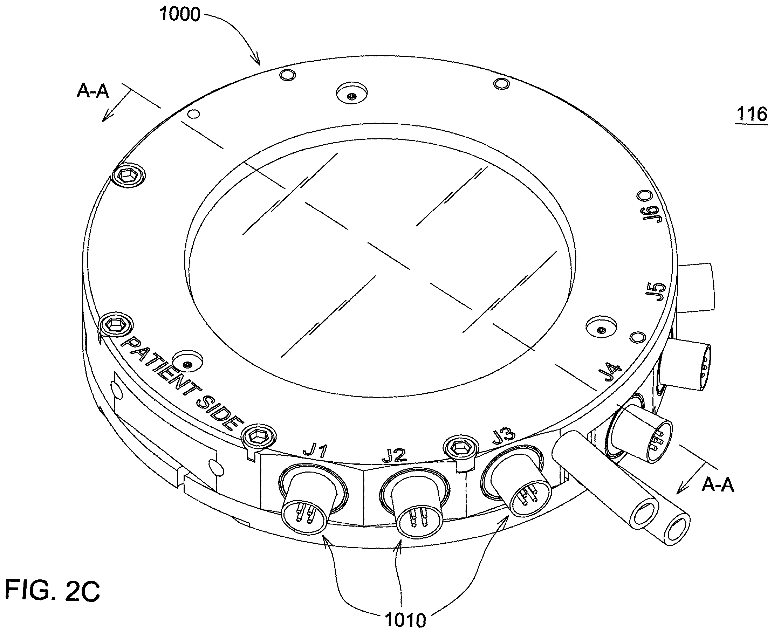

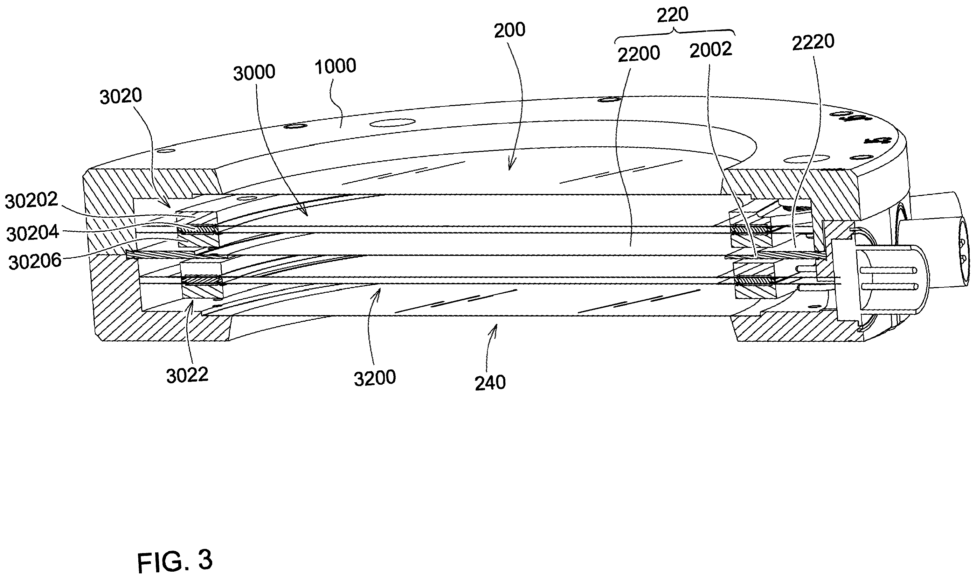

FIG. 2A is a plan view of a target side of an example embodiment of the ion chamber 116 shown in FIG. 1. FIG. 2B is a plan view of a patient side of the ion chamber 116 shown in FIG. 2A. FIG. 2C is a patient side perspective view of the ion chamber 116 shown in FIGS. 2A and 2B. FIG. 3 is a cross-sectional view of the ion chamber 116 along A-A in FIG. 2C.

Referring to FIGS. 2A-2C, the ion chamber 116 includes an ion chamber housing 1000 (also referred to as an ion chamber body or body assembly) providing an enclosed volume for internal components including, for example, electrode assemblies, clamping assemblies, support structures, and one or more ion chamber window assemblies. The housing 1000 may be hermetically sealed to enclose a gas such as air or other suitable gas which can be ionized by radiation. Alternatively, the housing 1000 may be non-hermetically sealed. The housing 1000 may be copper plated metal, such as aluminum or stainless steel. In some example embodiments, the entire housing 1000 may be copper plated. In other examples, however, only certain portions of the housing 1000 may be copper plated. For example, the portions of the housing 1000 to which plated ion chamber windows are secured and/or fixed (e.g., soldered) may be copper plated.

By way of example, the housing 1000 may include at least a target side metal part 1002 (also referred to as a target side housing part) and a patient side metal part 1004 (also referred to as a patient side housing part), which are configured to engage with, and be fixed to, each other to form the housing 1000.

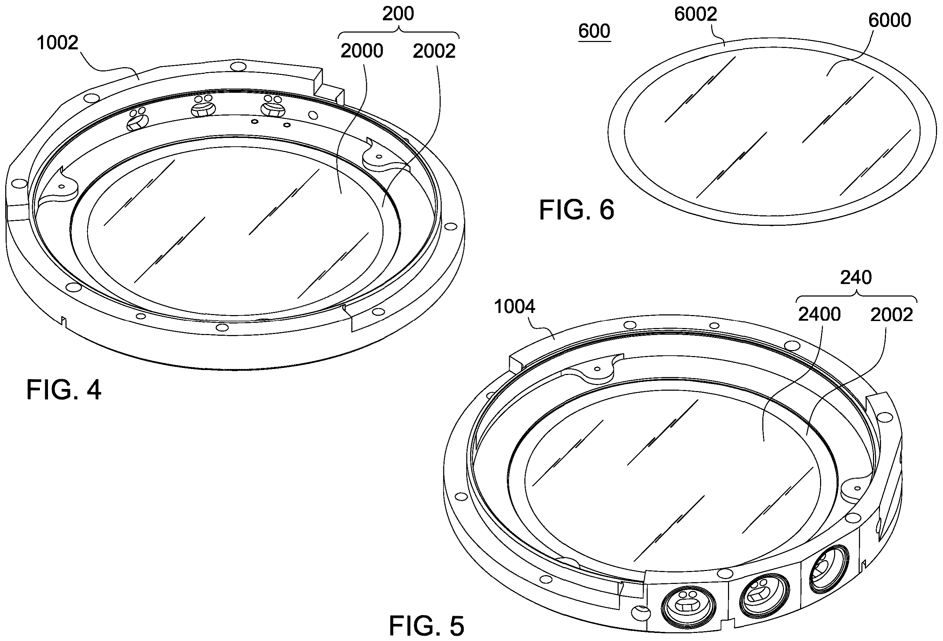

FIG. 4 is a perspective view of an inside of the target side metal part 1002 shown in FIG. 2A. FIG. 5 is a perspective view of an inside of the patient side metal part 1004 shown in FIG. 2B.

Referring to FIGS. 2A-2C, 4 and 5, the target side metal part 1002 and the patient side metal part 1004 are ring-shaped, with each part having a respective opening at the middle (or central) portion, region or area of the ring. When engaged with one another, the respective openings are at opposite ends of the ion chamber 116, and the abutting surfaces of the target side metal part 1002 and the patient side metal part 1004 may be sealed by any suitable mechanism to provide an airtight seal between the parts. The target side metal part 1002 and the patient side metal part 1004 may be fixed to one another via one or more bolts, screws, or the like. Although example embodiments are described with regard to a ring shape, example embodiments should not be limited to this example.

As shown in FIGS. 2A and 4, a target side ion chamber window assembly 200 (also referred to as a first or second ion chamber window assembly) is fixed across the opening of the target side metal part 1002. In at least one example embodiment, a peripheral edge of the ion chamber window assembly 200 may be fixed (e.g., permanently fixed) to an inner side of the target side metal part 1002. In one example, the peripheral edge of the ion chamber window assembly 200 may be soldered, welded or brazed to the inner side of the target side metal part 1002 to create an airtight seal.

As shown in FIG. 4, the ion chamber window assembly 200 includes an ion chamber window 2000 and a metal plating 2002 formed at a peripheral edge of the ion chamber window 2000. The ion chamber window 2000 may be formed of at least a first metal and the metal plating 2002 may be formed of a second metal. The first metal and the second metal may be different. The ion chamber window 2000 is exposed through the opening of the target side metal part 1002.

As shown in FIGS. 2B, 2C, and 5, a patient side ion chamber window assembly 240 (also referred to as a first or second ion chamber window assembly) is fixed across the opening of the patient side metal part 1004. In at least one example embodiment, a peripheral edge of the ion chamber window assembly 240 may be fixed (e.g., permanently fixed) to an inner side of the patient side metal part 1004. In one example, the peripheral edge of the ion chamber window assembly 240 may be soldered, welded or brazed to the inner side of the patient side metal part 1004 to create an airtight seal. As shown in FIG. 5, the ion chamber window assembly 240 includes an ion chamber window 2400 and metal plating 2002 formed at a peripheral edge of the ion chamber window 2400. The ion chamber window 2400 may be formed of at least the first metal and exposed through the opening of the target side metal part 1002.

As shown in FIG. 3, the ion chamber 116 further includes a central ion chamber window assembly 220 (also referred to as third ion chamber window assembly) arranged at a vertically central position between the ion chamber window assembly 200 and the ion chamber window assembly 240. The central ion chamber window assembly 220 is fixed (e.g., permanently fixed) to, and supported by, a central window support member 2220 (also referred to as a window support structure), which is further supported by the housing 1000. In one example, the peripheral edge of the ion chamber window assembly 220 may be soldered, welded or brazed to the central window support member 2220 to create an airtight seal.

The central window support member 2220 may be ring shaped and fit into a notch at the inner periphery of the housing 1000. In at least one example embodiment, a peripheral edge of the central ion chamber window assembly 220 may be fixed to the patient side of the central window support member 2220. However, example embodiments should not be limited to this example. As with the ion chamber window assemblies 200 and 240 discussed above, the central ion chamber window assembly 220 includes an ion chamber window 2200 and metal plating 2002 formed at a peripheral edge of the ion chamber window 2200. The ion chamber window 2200 may be formed of at least the first metal.

Still referring to FIG. 3, the ion chamber 116 further includes a first electrode assembly 3000 and a second electrode assembly 3200. The first electrode assembly 3000 is arranged between the ion chamber window assembly 200 and the central ion chamber window assembly 220. A first ring clamping assembly 3020 is provided to secure the first electrode assembly 3000 within the ion chamber 116.

The first ring clamping assembly 3020 includes an upper ring part 30202 and a lower ring part 30206 separated by a spacer ring 30204. The upper ring part 30202 is secured to the lower ring part 30206 via one or more screws, bolts, or the like. When secured, the upper ring part 30202 and the lower ring part 30206 clamp the spacer ring 30204 and the first electrode assembly 3000 to fix the components in place within the ion chamber 116.

A second electrode assembly 3200 is arranged between the ion chamber window assembly 240 and the central ion chamber window assembly 220. In this regard, the central ion chamber window assembly 220 is between the first electrode assembly 3000 and the second electrode assembly 3200. A second ring clamping assembly 3022 is provided to secure the second electrode assembly 3200 within the ion chamber 116. The second ring clamping assembly 3022 may be the same or substantially the same as the first ring clamping assembly 3020, and thus, a detailed discussion is omitted for the sake of brevity.

Each of the first electrode assembly 3000 and the second electrode assembly 3200 includes a high voltage electrode and a measurement or collection electrode spaced apart from each other by the spacer ring 30204. The collection electrode may include a pattern of a conductive material formed or deposited on an insulative film or plate. Therefore, the collection electrode may include a combination of electrodes patterned, segmented, or arranged for measuring different combinations of radiation passing through the ion chamber 116, thereby providing information about the profile of the radiation such as the intensity, the homogeneity of the beam across the treatment filed, the symmetry of the beam about the beam's central axis, the alignment and/or directionality of the beam, etc.

The ion chamber 116 further includes a plurality of terminals 1010 that electrically connect the electrodes within the housing 1000. The terminals 1010 provide working voltage to the electrodes and output signals to electrical circuitry (e.g., the control system 120) for measurement and/or further analysis.

FIG. 6 is a perspective view of an example embodiment of a metal ion chamber window assembly 600. At least one or all of the metal ion chamber window assemblies 200, 220 and 240 may be the same or substantially the same as the metal ion chamber window assembly 600 shown in FIG. 6.

Referring to FIG. 6, the metal ion chamber window assembly 600 includes an ion chamber window 6000 formed of a first metal. In one example, the ion chamber window 6000 may be about 0.0020" (50 um) thick and/or formed of aluminum. In one example, the aluminum may be 5052 H19 aluminum. The ion chamber window 6000, prior to being plated, may be laser cut to avoid deformation (e.g., wrinkling) of the aluminum or other thin metal foil utilized to form the ion chamber window.

A metal plating 6002 is provided at a periphery of the ion chamber window 6000. The metal plating 6002 may be formed of a second metal, such as nickel (Ni) to enable soldering of the ion chamber window assembly 600 to the inner surface of the housing 1000 as discussed herein. In at least one example embodiment, nickel sulfamate (Ni(SO₃NH₂)₂) may be used to create a layer of pure nickel as the metal plating 6002. The metal plating 6002 may be formed to a thickness of about 300-400 micro inches. In another example, the metal plating 6002 may be copper (Cu) plating, tin plating, gold plating, or any other readily solderable metal. The metal plating 6002 may be formed on the ion chamber window 6000 using any known method including, for example, sputtering, electroplating, electroless plating, physical vapor deposition (PVD), plasma spray coating, or the like.

In the example embodiment shown in FIG. 6, the metal plating 6002 is formed only at the periphery of the ion chamber window 6000. The metal plating 6002 is not formed at a central portion, region or area of the ion chamber window 6000 nor is the metal plating 6002 formed within the path of the beam through the ion chamber 116 once installed in a radiation apparatus such as the radiation apparatus 100 shown in FIG. 1. When plating the ion chamber window 6000, the central portion, region or area may be masked so the area of the window within the beam path is unaltered and/or the metal plating is formed only at the periphery of the window.

One or more example embodiments provide ion chambers having metal windows, which replace conventional polyimide (e.g., Kapton^{®}) windows. By utilizing metal windows, supply chain stability may be improved while remaining cost neutral relative to conventional ion chambers. Use of metal ion chamber windows may also improve cleaning and cleaning consistency, improve electron scattering profiles, and/or reduced window bow enabling larger volume ion chambers.

Illustrative embodiment 1. An ion chamber, comprising: an ion chamber housing having a first opening; a first ion chamber window assembly including a first ion chamber window and a first metal plating arranged at a peripheral edge of the first ion chamber window, the first ion chamber window being formed of at least a first metal and having a first surface exposed through the first opening, and the first metal plating being formed of a second metal, wherein the first metal is different from the second metal, and the first ion chamber window assembly is fixed, via the first metal plating, on an inner side of the ion chamber housing at a periphery of the first opening.

Illustrative embodiment 2. The ion chamber of illustrative embodiment 1, wherein the first metal is aluminum.

Illustrative embodiment 3. The ion chamber of illustrative embodiment s2, wherein the aluminum is 5052 H19 aluminum.

Illustrative embodiment 4. The ion chamber of any of illustrative embodiments 1-3, wherein the first metal plating is a nickel plating.

Illustrative embodiment 5. The ion chamber of any of illustrative embodiments 1-4, wherein the first metal plating is formed only at the peripheral edge of the first ion chamber window.

Illustrative embodiment 6. The ion chamber of any of illustrative embodiments 1-5, wherein the first metal plating is outside a beam path through the ion chamber.

Illustrative embodiment 7. The ion chamber of any of illustrative embodiments 1-6, wherein the first metal plating is soldered to the inner side of the ion chamber housing.

Illustrative embodiment 8. The ion chamber of any of illustrative embodiments 1-7, wherein: the ion chamber housing includes a first part having the first opening and a second part having a second opening; the first part and the second part are configured to be fixed to one another to form the ion chamber housing; the first opening and the second opening are at opposite ends of the ion chamber; the first ion chamber window assembly is fixed to an inner side of the first part of the ion chamber housing; the ion chamber includes a second ion chamber window assembly fixed on an inner side of the second part of the ion chamber housing; and a second surface of the second ion chamber window assembly is exposed through the second opening.

Illustrative embodiment 9. The ion chamber of illustrative embodiment 8, wherein: the second ion chamber window assembly includes a second ion chamber window and a second metal plating arranged at a peripheral edge of the second ion chamber window; the second ion chamber window is formed of at least the first metal; the second metal plating is formed of the second metal; and the second ion chamber window assembly is fixed, via the second metal plating, on the inner side of the second part of the ion chamber housing.

Illustrative embodiment 10. The ion chamber of any of illustrative embodiments 8 or 9, further comprising: a third ion chamber window assembly arranged within the ion chamber housing between the first ion chamber window assembly and the second ion chamber window assembly; and a window support structure fixed to an inner part of the ion chamber housing, the window support structure configured to support the third ion chamber window assembly.

Illustrative embodiment 11. The ion chamber of illustrative embodiment 10, wherein: the third ion chamber window assembly includes a third ion chamber window and a third metal plating at a peripheral edge of the third ion chamber window; the third ion chamber window is formed of at least the first metal; and the third metal plating is formed of the second metal.

Illustrative embodiment 12. The ion chamber of illustrative embodiment 11, wherein the third metal plating is soldered to the window support structure.

Illustrative embodiment 13. The ion chamber of any of illustrative embodiments 10-12, further comprising: a first electrode assembly arranged between the first ion chamber window assembly and the third ion chamber window assembly; and a second electrode assembly arranged between the second ion chamber window assembly and the third ion chamber window assembly, wherein the third ion chamber window assembly is between the first electrode assembly and the second electrode assembly.

Illustrative embodiment 14. A radiation apparatus comprising: a radiation source configured to emit a radiation beam toward a patient; a radiation beam adjustment structure configured to adjust one or more characteristics of the radiation beam; and an ion chamber arranged in a path of the radiation beam, the ion chamber configured to monitor one or more characteristics of the radiation beam. The ion chamber includes: an ion chamber housing having a first opening; a first ion chamber window assembly including a first ion chamber window and a first metal plating arranged at a peripheral edge of the first ion chamber window, the first ion chamber window being formed of at least a first metal and having a first surface exposed through the first opening, and the first metal plating being formed of a second metal; wherein the first metal is different from the second metal, and the first ion chamber window assembly is fixed, via the first metal plating, on an inner side of the ion chamber housing at a periphery of the first opening.

Illustrative embodiment 15. The radiation apparatus of illustrative embodiment 14, wherein the first metal is aluminum.

Illustrative embodiment 16. The radiation apparatus of illustrative embodiment 15, wherein the aluminum is 5052 H19 aluminum.

Illustrative embodiment 17. The radiation apparatus of illustrative embodiment 15, wherein the first metal plating is a nickel plating.

Illustrative embodiment 18. The radiation apparatus of illustrative embodiment 14, wherein the first metal plating is formed only at the peripheral edge of the first ion chamber window.

Illustrative embodiment 19. The radiation apparatus of illustrative embodiment 14, wherein the first metal plating is soldered to the inner side of the ion chamber housing.

Illustrative embodiment 20. The radiation apparatus of illustrative embodiment 14, wherein: the ion chamber housing includes a first part having the first opening and a second part having a second opening; the first part and the second part are configured to be fixed to one another to form the ion chamber housing; the first opening and the second opening are at opposite ends of the ion chamber; the first ion chamber window assembly is fixed to an inner side of the first part of the ion chamber housing; and the ion chamber includes a second ion chamber window assembly fixed on an inner side of the second part of the ion chamber housing, wherein a second surface of the second ion chamber window assembly is exposed through the second opening, a third ion chamber window assembly arranged within the ion chamber housing between the first ion chamber window assembly and the second ion chamber window assembly, and a window support structure fixed to an inner part of the ion chamber housing, the window support structure configured to support the third ion chamber window assembly.

Although the terms first, second, etc. may be used herein to describe various elements, these elements should not be limited by these terms. These terms are only used to distinguish one element from another. For example, a first element could be termed a second element, and similarly, a second element could be termed a first element, without departing from the scope of this disclosure. As used herein, the term "and/or," includes any and all combinations of one or more of the associated listed items.

When an element is referred to as being "connected," or "coupled," to another element, it can be directly connected or coupled to the other element or intervening elements may be present. By contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific details are provided in the following description to provide a thorough understanding of example embodiments. However, it will be understood by one of ordinary skill in the art that example embodiments may be practiced without these specific details. For example, systems may be shown in block diagrams so as not to obscure the example embodiments in unnecessary detail. In other instances, well-known processes, structures and techniques may be shown without unnecessary detail in order to avoid obscuring example embodiments.

As discussed herein, illustrative embodiments will be described with reference to acts and symbolic representations of operations (e.g., in the form of flow charts, flow diagrams, data flow diagrams, structure diagrams, block diagrams, etc.) that may be implemented as program modules or functional processes include routines, programs, objects, components, data structures, etc., that perform particular tasks or implement particular abstract data types and may be implemented using existing hardware, for example, processing or control circuitry such as, but not limited to, one or more processors, one or more Central Processing Units (CPUs), one or more controllers, one or more arithmetic logic units (ALUs), one or more digital signal processors (DSPs), one or more microcomputers, one or more field programmable gate arrays (FPGAs), one or more System-on-Chips (SoCs), one or more programmable logic units (PLUs), one or more microprocessors, one or more Application Specific Integrated Circuits (ASICs), or any other device or devices capable of responding to and executing instructions in a defined manner.

Although a flow chart may describe the operations as a sequential process, many of the operations may be performed in parallel, concurrently or simultaneously. In addition, the order of the operations may be re-arranged. A process may be terminated when its operations are completed, but may also have additional steps not included in the figure. A process may correspond to a method, function, procedure, subroutine, subprogram, etc. When a process corresponds to a function, its termination may correspond to a return of the function to the calling function or the main function.

As disclosed herein, the term "memory," "storage medium," "processor readable medium," "computer readable storage medium" or "non-transitory computer readable storage medium" may represent one or more devices for storing data, including read only memory (ROM), random access memory (RAM), magnetic RAM, core memory, magnetic disk storage mediums, optical storage mediums, flash memory devices and/or other tangible machine-readable mediums for storing information. The term "computer-readable medium" may include, but is not limited to, portable or fixed storage devices, optical storage devices, and various other mediums capable of storing, containing or carrying instruction(s) and/or data.

Furthermore, example embodiments may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a computer readable storage medium. When implemented in software, a processor or processors will perform the necessary tasks. For example, as mentioned above, according to one or more example embodiments, at least one memory may include or store computer program code, and the at least one memory and the computer program code may be configured to, with at least one processor, cause a network element or network device to perform the necessary tasks. Additionally, the processor, memory and example algorithms, encoded as computer program code, serve as means for providing or causing performance of operations discussed herein.

The terms "including" and/or "having," as used herein, are defined as comprising (i.e., open language). The term "coupled," as used herein, is defined as connected, although not necessarily directly, and not necessarily mechanically. Terminology derived from the word "indicating" (e.g., "indicates" and "indication") is intended to encompass all the various techniques available for communicating or referencing the object/information being indicated. Some, but not all, examples of techniques available for communicating or referencing the object/information being indicated include the conveyance of the object/information being indicated, the conveyance of an identifier of the object/information being indicated, the conveyance of information used to generate the object/information being indicated, the conveyance of some part or portion of the object/information being indicated, the conveyance of some derivation of the object/information being indicated, and the conveyance of some symbol representing the object/information being indicated.

According to example embodiments, medical systems, may be (or include) hardware, firmware, hardware executing software or any combination thereof. Such hardware may include processing or control circuitry such as, but not limited to, one or more processors, one or more CPUs, one or more controllers, one or more ALUs, one or more DSPs, one or more microcomputers, one or more FPGAs, one or more SoCs, one or more PLUs, one or more microprocessors, one or more ASICs, or any other device or devices capable of responding to and executing instructions in a defined manner.

Benefits, other advantages, and solutions to problems have been described above with regard to specific embodiments. However, the benefits, advantages, solutions to problems, and any element(s) that may cause or result in such benefits, advantages, or solutions, or cause such benefits, advantages, or solutions to become more pronounced are not to be construed as a critical, required, or essential feature or element of any or all the claims.

## Claims

1. An ion chamber (116), comprising:
an ion chamber housing (1000) having a first opening; and
a first ion chamber window assembly (200, 600) including a first ion chamber window (2000, 6000) and a first metal plating (2002, 6002) arranged at a peripheral edge of the first ion chamber window, the first ion chamber window (2000, 6000) being formed of at least a first metal and having a first surface exposed through the first opening, and the first metal plating (2002, 6002) being formed of a second metal, wherein
the first metal is different from the second metal, and
the first ion chamber window assembly (200, 600) is fixed, via the first metal plating (2002, 6002), on an inner side of the ion chamber housing at a periphery of the first opening.

2. The ion chamber of claim 1, wherein the first metal is aluminum, and optionally the aluminum is 5052 H19 aluminum.

3. The ion chamber of claim 1 or 2, wherein the first metal plating is a nickel plating.

4. The ion chamber of claim 1, 2 or 3, wherein the first metal plating is formed only at the peripheral edge of the first ion chamber window, and optionally the first metal plating is outside a beam path through the ion chamber.

5. The ion chamber of any one of the preceding claims, wherein the first metal plating is soldered to the inner side of the ion chamber housing.

6. The ion chamber of any one of the preceding claims, wherein
the ion chamber housing (1000) includes a first part having the first opening and a second part having a second opening,
the first part and the second part are configured to be fixed to one another to form the ion chamber housing,
the first opening and the second opening are at opposite ends of the ion chamber,
the first ion chamber window assembly is fixed to an inner side of the first part of the ion chamber housing,
the ion chamber includes a second ion chamber window assembly fixed on an inner side of the second part of the ion chamber housing, and
a second surface of the second ion chamber window assembly is exposed through the second opening, and optionally the second ion chamber window assembly includes a second ion chamber window and a second metal plating arranged at a peripheral edge of the second ion chamber window,
the second ion chamber window is formed of at least the first metal,
the second metal plating is formed of the second metal, and
the second ion chamber window assembly is fixed, via the second metal plating, on the inner side of the second part of the ion chamber housing.

7. The ion chamber of claim 6, further comprising:
a third ion chamber window assembly arranged within the ion chamber housing between the first ion chamber window assembly and the second ion chamber window assembly, and
a window support structure fixed to an inner part of the ion chamber housing, the window support structure configured to support the third ion chamber window assembly, and optionally,
the third ion chamber window assembly includes a third ion chamber window and a third metal plating at a peripheral edge of the third ion chamber window,
the third ion chamber window is formed of at least the first metal, and
the third metal plating is formed of the second metal.

8. The ion chamber of claim 7, wherein the third metal plating is soldered to the window support structure.

9. The ion chamber of claim 7 or 8, further comprising:
a first electrode assembly (3000) arranged between the first ion chamber window assembly and the third ion chamber window assembly; and
a second electrode assembly (3200) arranged between the second ion chamber window assembly and the third ion chamber window assembly, wherein
the third ion chamber window assembly is between the first electrode assembly and the second electrode assembly.

10. A radiation apparatus (100) comprising:
a radiation source (102) configured to emit a radiation beam (104, 112) toward a patient (110);
a radiation beam adjustment structure (106, 107, 108, 114) configured to adjust one or more characteristics of the radiation beam; and
an ion chamber (116) arranged in a path of the radiation beam, the ion chamber configured to monitor one or more characteristics of the radiation beam, and the ion chamber including
an ion chamber housing (1000) having a first opening,
a first ion chamber window assembly (200) including a first ion chamber window (2000, 6000) and a first metal plating (2002, 6002) arranged at a peripheral edge of the first ion chamber window (2000, 6000), the first ion chamber window (200) being formed of at least a first metal and having a first surface exposed through the first opening, and the first metal plating (2002, 6002) being formed of a second metal, wherein
the first metal is different from the second metal, and
the first ion chamber window assembly is fixed, via the first metal plating, on an inner side of the ion chamber housing at a periphery of the first opening.

11. The radiation apparatus of claim 10, wherein the first metal is aluminum, and optionally, the aluminum is 5052 H19 aluminum.

12. The radiation apparatus of claim 10 or 11, wherein the first metal plating is a nickel plating.

13. The radiation apparatus of claim 10, 11 or 12, wherein the first metal plating is formed only at the peripheral edge of the first ion chamber window.

14. The radiation apparatus of any one of claims 10-13, wherein the first metal plating is soldered to the inner side of the ion chamber housing.

15. The radiation apparatus of any one of claims 10-14, wherein
the ion chamber housing (1000) includes a first part having the first opening and a second part having a second opening,
the first part and the second part are configured to be fixed to one another to form the ion chamber housing,
the first opening and the second opening are at opposite ends of the ion chamber (116),
the first ion chamber window assembly (220, 240, 600) is fixed to an inner side of the first part of the ion chamber housing, and
the ion chamber includes
a second ion chamber window assembly (220, 240, 600) fixed on an inner side of the second part of the ion chamber housing, wherein a second surface of the second ion chamber window assembly is exposed through the second opening,
a third ion chamber window assembly (220, 240, 600) arranged within the ion chamber housing between the first ion chamber window assembly and the second ion chamber window assembly, and
a window support structure fixed to an inner part of the ion chamber housing, the window support structure configured to support the third ion chamber window assembly.
